# FASCICULE DE BREVET EUROPEEN

(11) **EP 3 250 295 B1**
(45) Date de publication et mention de la délivrance du brevet: **15.07.2020**
(21) Numéro de dépôt: 16707196.8
(22) Date de dépôt: 28.01.2016
(51) Int. Cl.: A61K 8/9789, A61K 8/9794, A61Q 19/08, A61Q 19/00, A61K 36/21, A61K 36/886, A61K 36/896, C12N 5/04

(54) **PROCEDE DE PREPARATION D'UNE COMPOSITION TOPIQUE A BASE DE CELLULES VÉGÉTALES DÉDIFFÉRENTIÉES ET ÉLICITÉES EN CULTURE IN VITRO**
HERSTELLUNGSVERFAHREN EINER TOPISCHEN ZUSAMMENSETZUNG DEDIFFERENZIERTEN UND IN VITRO ANGEREGTEN PFLANZENZELLEN
METHOD OF MANUFACTURING A TOPICAL COMPOSITION COMPRISING DEDIFFERENTIATED PLANT CELLS ELICITED IN IN VITRO CULTURE

(30) Priorité: 30.01.2015 EP 15000285
(43) Date de publication de la demande: 06.12.2017
(73) Titulaire: Naolys Sarl, 33610 Cestas Pierroton (FR)
(72) Inventeur: ENNAMANY, Rachid, F-33800 Bordeaux (FR)
(74) Mandataire: Powis de Tenbossche, Roland
(86) Numéro de dépôt international: PCT/IB2016/000058
(87) Numéro de publication internationale: WO 2016/120713

(56) Documents cités:
- WO-A2-03/077881
- WO-A2-2013/033365
- JP-A- 2002 193 820
- US-A- 4 241 536
- MATKOWSKI ET AL: "Plant in vitro culture for the production of antioxidants - A review", BIOTECHNOLOGY ADVANCES, ELSEVIER PUBLISHING, BARKING, GB, vol. 26, no. 6, 1 novembre 2008 (2008-11-01), pages 548-560, XP027181985, ISSN: 0734-9750 [extrait le 2008-07-16]
- ANTOGNONI ET AL: "Induction of flavonoid production by UV-B radiation in Passiflora quadrangularis callus cultures", FITOTERAPIA, IDB HOLDING, MILAN, IT, vol. 78, no. 5, 1 juillet 2007 (2007-07-01), pages 345-352, XP022143510, ISSN: 0367-326X, DOI: 10.1016/J.FITOTE.2007.02.001
- GOGOBERIDZE M K ET AL: "Steroid substances in the Yucca gloriosa L. cell and tissue culture and their formation during morphogenesis", PLANT SCIENCE, ELSEVIER IRELAND LTD, IE, vol. 84, no. 2, 1 janvier 1992 (1992-01-01), pages 201-207, XP025447717, ISSN: 0168-9452, DOI: 10.1016/0168-9452(92)90135-9 [extrait le 1992-01-01]

## Description

Cette demande de brevet internationale revendique la priorité de la demande de brevet européenne 15 000 285.5 déposée le 30 janvier 2015 au nom de ENNAMANY Rachid.

L'invention a pour objet une composition à usage topique, en particulier cosmétique, riche en métabolites produits par des cellules végétales provenant de plantes particulières. En particulier, l'invention a pour objet une composition contenant des cellules végétales dédifférenciées et élicitées puis séchées partiellement ou totalement de préférence, lyophilisées, et ensuite dispersées dans cette composition.

Par cellules végétales dédifférenciées, on entend toute cellule végétale ne présentant aucun des caractères d'une spécialisation particulière et capable de vivre par elle-même et non en dépendance avec d'autres cellules.

Les cellules végétales dédifférenciées peuvent être obtenues à partir de matériel végétal issu de plante entière ou de partie de plante comme les feuilles, les tiges, les fleurs, les pétales, les racines, les fruits, leur peau, l'enveloppe les protégeant, les graines, les anthères, la sève, les épines, les bourgeons, l'écorce, les baies, et des mélanges de ceux-ci.

Préférentiellement, les cellules végétales dédifférenciées sont obtenues à partir d'écorce, de feuilles, de bourgeons et de la peau des fruits.
Les cellules végétales dédifférenciées utilisables selon l'invention peuvent être obtenues à partir de végétaux obtenus par culture in vivo ou issu de culture in vitro.

Par culture in vivo on entend toute culture de type classique c'est à dire en sol à l'air libre ou en serre ou encore hors sol ou en milieu hydroponique.

Par culture in vitro, on entend l'ensemble des techniques connues de l'homme du métier qui permet de manière artificielle l'obtention d'un végétal ou d'une partie d'un végétal. La pression de sélection imposée par les conditions physico-chimiques lors de la croissance des cellules végétales in vitro permet d'obtenir un matériel végétal standardisé, exempt de contaminations et disponible tout au long de l'année contrairement aux plantes cultivées in vivo.
Préférentiellement selon l'invention on utilise des cellules végétales dédifférenciées issues de culture in vitro.
Les cellules végétales dédifférenciées utilisables selon l'invention peuvent être obtenues par toute méthode connue de l'art antérieur. A cet égard on peut citer les méthodes décrites par E.F. George et P.D. Sherrington dans Plant Propagation by tissue culture, handbook and directory of commercial laboratories (Exegetics Ltd 1984).
Les milieux de culture utilisables selon l'invention sont ceux généralement connus de l'homme du métier. On peut citer à titre d'exemples les milieux de Gamborg, Murashige et Skoog, Heller, White etc.... On trouvera dans "Plant Culture Média : formulations and uses" de E.F. George, DJM Puttock et H.J George (Exegetics Ltd 1987, tome 1 & 2) des descriptions complètes de ces milieux.

Préférentiellement selon l'invention on prépare les cellules végétales dédifférenciées cultivées sur milieu de Murashige et Skoog.

### Etat de la technique

On connaît par le document FR 2795637 une composition cosmétique contenant un extrait de cellules végétales dédifférenciées pour éviter les problèmes d'odeur. Cette composition contient un extrait de cellules végétales dédifférenciées mais non élicitées, de sorte que cette composition est pauvre en métabolites secondaires ou en phytoalexines, voire est sensiblement exempte de tels composés. De plus ce document décrit l'utilisation d'extrait aqueux obtenus après broyage des cellules dans leur milieu de culture puis élimination des particules en suspension avec une perte inévitable des métabolites liés aux particules en suspension. Afin d'éliminer les protéases et notamment les oxydases ce document préconise également l'emploi de filtres capturant les molécules d'un poids moléculaire supérieur à 100.000 daltons perdant ainsi dans l'extrait final tous les métabolites supérieurs à ce poids et qui peuvent s'avérer d'un grand intérêt pour l'industrie cosmétique. De plus afin d'éliminer les problèmes dus à l'oxydation le document préconise l'ajout de stabilisants, notamment la cystèine et/ ou des dérivés soufrés ce qui entraîne nécessairement une pureté moindre de l'extrait avec des étapes subséquentes de filtration. Les méthodes décrites dans ce document nécessitent la mise en œuvre de moyens compliqués pour l'obtention d'extraits dont, tant la pureté (nombreux additifs), que la qualité et la concentration (en métabolites) n'est pas optimale. De plus les nombreuses étapes nécessaires à l'obtention des extraits de ce procédé induisent des coûts élevés et le risque de contaminations de part les nombreuses manipulations et additifs.

On connaît les cultures de cellules dédifférenciées, d'autre part on connaît les mécanismes d'élicitation de ces cellules suivi d'étapes d'extractions et de filtrations diverses suivis de lyophilisation afin d'incorporer les extraits obtenus dans une préparation cosmétique ou pharmaceutique. De tels procédés sont par exemple décrits dans US 4,241,536 ; EP 378 921, WO 88/00968, EP 1 203 811, etc. pour des espèces de plantes diverses. Le contenu de ces documents est incorporé dans la présente description par référence pour décrire des milieux de culture, des espèces de plantes, des éliciteurs possibles, etc.

De nos jours, malgré les compétences et le savoir faire des industries dans le domaine de l'extraction végétale, et malgré les progrès de la chimie organique, plusieurs étapes d'extraction sont indispensables pour obtenir une matière première végétale.

Plusieurs inconvénients sont imputés à ces extractions :
- perte de la structure tertiaire des molécules isolées,
- présence des différents solvants au niveau du produit fini,
- hétérogénéité des substrats nécessitant des extractions fines faisant appel à des solvants de plus en plus toxiques,
- qualité de l'extrait en fonction de l'état physiologique de la plante au moment de la récolte,
- production de l'extrait limitée en fonction des saisons,

Face à ces facteurs limitants et au regain d'intérêt des consommateurs pour tout ce qui est d'origine naturelle, plusieurs tentatives d'obtention de cellules ont été réalisées. Ainsi, à ce jour, deux procédés majeurs ont été exploités :
- La culture de cellules à partir d'organismes unicellulaires ou de microorganismes, technique peu originale se basant sur la reproduction des conditions de vie normale. Cependant ces organismes sont primitifs et ne développent pas de métabolisme secondaire, source des principes actifs les plus intéressants.
- L'obtention des cellules à partir de fruits (cellules fraîches) après digestion enzymatique. Les limites de ce procédé résident dans le fait que les fruits ne sont pas aseptiques et qu'ils peuvent contenir des résidus de pesticides (fongicides, herbicides, insecticides,...). D'autre part, les enzymes (cellulases, pectinases,...) utilisées en quantité importante (2%, P/P) pour la digestion des parois végétales et l'obtention des cellules sans paroi (protoplastes) se retrouvent dans le produit fini. Par ailleurs, les enzymes utilisées peuvent altérer la qualité des métabolites. Enfin, l'utilisation de cette technique permet seulement de récupérer des protoplastes (cellules sans paroi cellulaire), structures fragiles ne pouvant pas orienter leur métabolisme.

On connaît également par le document WO 03/077880 un procédé de préparation d'un broyat de cellules dédifférentiées et élicitées pour produire des phytoalexines. Dans ce document, les cellules végétales sont sélectionnées dans des familles particulières et sont élicitées selon des étapes particulières.

Le document « Plant in vitro culture for the production of antioxidants - A review » , MATKOWSKI A., Biotechnology Advances 26(2008) 548-560 décrit un procédé de culture in vitro pour la production d'antioxydants. Selon ce document, des stratégies diverses existent pour accroître la production de métabolites, à savoir les conditions de culture, avec l'irradiation comme facteur favorisant la biosynthèse ; la sélection de cellules hautement productives, ; l'élicitation et le stress (voir page 556 : « Irradiation with UV has been also used for making the plant cells synthetsize more antioxidants ... The irradiation stimulated a large increase in the amount of flavanoid... » Dans sa conclusion, ce document indique : « Despite the imense variety of antioxidant compounds obtained from the in vitro cultured plant, their cells, tissues and organs, not many examples of pratically applicable technologies can be named ..."

Le document « Induction of flavanoid production by UV-B radiation in Passiflora quadrangularis callus cultures », Antagoni F. et al, Fitoterapia 78(207) 345 - 352, enseigne la production de flavanoïdes par irradiation UV-B dans des cultures de Passiflora quadrangularis. Ainsi que stipulé en page 346, « Unfortunately the yield of the desired end-product is often too low to make this viable alternative to extraction from field-grown plants. Consequently, many studies have been undertaken to unravel biosynthetic pathways and understand their régulation ... »

Les inventeurs ont maintenant découvert qu'en soumettant des cellules dédifférentiées de familles de plantes végétales particulières à un cycle d'élicitation particulier, il était possible d'obtenir des cellules contenant un mélange de molécules dans des proportions relatives entre elles qui permettaient d'assurer un traitement adéquat de problèmes liés à la peau.

Dans le présent mémoire, on entend par luminosité, un éclairage avec des rayons de longueur d'onde comprise entre 100 nm et 700nm, en particulier dans le spectre visible, et de préférence dans la gamme de 400 nm à 520 nm (correspondant au spectre de couleur bleu et vert. Lors des étapes d'éclairage, l'éclairage ne comprend avantageusement sensiblement pas de rayons de longueur d'onde inférieure à 100nm et/ou supérieure à 700nm. De préférence, plus de 90% des rayons, en particulier plus de 95% des rayons ont une longueur d'onde comprise entre 100 nm et 700 nm, de préférence comprise entre 400nm et 520 nm.

Dans le présent mémoire, les périodes de non luminosité sont avantageusement des périodes où le milieu de culture n'est sensiblement pas soumis à un rayonnement de longueur d'onde comprise entre 100nm et 700 nm, mais également de préférence inférieure à 100nm et supérieure à 700nm. Les périodes de non luminosité sont de préférence des périodes d'obscurité totale ou sensiblement totale.

L'invention a pour objet un procédé de préparation d'une composition à usage topique tel que revendiqué à la revendication 1, ce procédé étant essentiellement caractérisé en ce que l'on met des cellules végétales dédifférenciées d'une espèce choisie parmi le groupe constitué des espèces des familles suivantes : Agavaceae (en particulier les espèces: Agave, Beschorneria, Chlorophytum, Furcraea, Hesperaloe, Hesperoyucca, Yucca), Amaryllidaceae (en particulier l'espèce: Boophane, Brunsvigia, Cyrtanthus, Haemanthus, Rauhia), Anacardiaceae (en particulier l'espèce: Operculicarya, Pachycormus), Apiaceae (en particulier l'espèce: Steganotaenia), Apocynaceae (en particulier l'espèce: Adenium, Mandevilla, Pachypodium, Plumeria), Araceae (en particulier les espèces: Zamioculcas zamiifolia); Araliaceae (en particulier les espèces: Cussonia), Asclepiadaceae (en particulier les espèces: Absolmsia, Asclepias, Aspidoglossum, Aspidonepsis, Baynesia, Brachystelma, Caralluma, Ceropegia, Cibirhiza, Cynanchum, Dischidia, Dischidiopsis, Duvalia, Duvaliandra, Echidnopsis, Edithcolea, Fanninia, Fockea, Glossostelma, Hoodia, Hoya, Huernia, Huerniopsis, Ischnolepis, Larryleachia, Lavrania, Madangia, Marsdenia, Matelea, Micholitzia, Miraglossum, Notechidnopsis, Odontostelma, Ophionella, Orbea, Orbeanthus, Pachycarpus, Pectinaria, Petopentia, Piaranthus, Pseudolithos, Quaqua, Raphionacme, Rhytidocaulon, Riocreuxia, Sarcorrhiza, Sarcostemma, Schizoglossum, Schlechterella, Stapelia, Stapelianthus, Stapeliopsis, Stathmostelma, Stenostelma, Stomatostemma, Tavaresia, Trachycalymma, Tridentea, Tromotriche, White-sloanea, Xysmalobium), Asparagaceae (en particulier les espèces: Myrsiphyllum), Asphodelaceae ((en particulier les espèces: Aloe, Astroloba, Bulbine, Chortolirion, Gasteria, Haworthia, Poellnitzia, Trachyandra), Asteraceae (en particulier les espèces: Baeriopsis, Coulterella, Crassocephalum, Didelta, Gynura, Osteospermum, Othonna, Polyachyrus, Pteronia, Senecio), Balsaminaceae (en particulier les espèces: Impatiens), Basellaceae (en particulier les espèces: Anredera, Basella), Begoniaceae (en particulier les espèces: Begonia), Bombacaceae (en particulier les espèces: Adansonia, Cavanillesia, Ceiba, Pseudobombax), Brassicaceae (en particulier les espèces: Heliophila, Lepidium), Bromeliaceae, Burseraceae (en particulier les espèces: Beiselia, Bursea, Commiphora), Cactaceae (en particulier les espèces: Acanthocalycium, Acanthocereus, Ariocarpus, Armatocereus, Arrojadoa, Arthrocereus, Astrophytum, Austrocactus, Aztekium, Bergerocactus, Blossfeldia, Brachycereus, Browningia, Brasilicereus, Calymmanthium, Carnegiea, Cephalocereus, Cephalocleistocactus, Cereus, Cintia, Cipocereus, Cleistocactus, Coleocephalocereus, Copiapoa, Corryocactus, Coryphantha, Dendrocereus, Denmoza, Discocactus, Disocactus, Echinocactus, Echinocereus, Echinopsis, Epiphyllum, Epithelantha, Eriosyce, Escobaria, Escontria, Espostoa, Espostoopsis, Eulychnia, Facheiroa, Ferocactus, Frailea, Geohintonia, Gymnocalycium, Haageocereus, Harrisia, Hatiora, Hylocereus, Jasminocereus, Lasiocereus, Leocereus, Lepismium, Leptocereus, Leuchtenbergia, Lophophora, Maihuenia, Malacocarpus, Mammillaria, Mammilloydia, Matucana, Melocactus, Micranthocereus, Mila, Monvillea, Myrtillocactus, Neobuxbaumia, Neolloydia, Neoraimondia, Neowerdermannia, Obregonia, Opuntia, Oreocereus, Oroya, Ortegocactus, Pachycereus, Parodia, Pediocactus, Pelecyphora, Peniocereus, Pereskia, Pereskiopsis, Pilosocereus, Polaskia, Praecereus, Pseudoacanthocereus, Pseudorhipsalis, Pterocactus, Pygmaeocereus, Quiabentia, Rauhocereus, Rebutia, Rhipsalis, Samaipaticereus, Schlumbergera, Sclerocactus, Selenicereus, Stenocactus, Stenocereus, Stephanocereus, Stetsonia, Strombocactus, Tacinga, Thelocactus, Turbinicarpus, Uebelmannia, Weberbauerocereus, Weberocereus, Yungasocereus), Campanulaceae (en particulier les espèces: Brighamia), Capparidaceae (en particulier les espèces: Maerua), Caricaceae (en particulier les espèces: Carica, Jacarathia), Chenopodiaceae, Cochlospermaceae, Commelinaceae (en particulier les espèces: Aneilema, Callisia, Cyanotis, Tradescantia, Tripogandra), Convolvulaceae (en particulier les espèces: Ipomea, Sictocardia, Turbina), Crassulaceae (en particulier les espèces: Adromischus, Aeonium, Afrovivella, Aichryson, Cotyledon, Crassula, Cremnophila, Cremnosedum, Dudleya, Echeveria, Graptopetalum, Hylotelephium, Hypagophytum, Kalanchoe, Lenophyllum, Meterostachys, Monanthes, Orostachys, Pachyphytum, Perrierosedum, Phedimus, Pistorinia, Prometheum, Pseudosedum, Rhodiola, Rosularia, Sedella, Sedum, Sempervivum, Sinocrassula, Thompsonella, Tylecodon, Umbilicus, Villadia), Cucurbitaceae (en particulier les espèces: Apodanthera, Brandegea, Cephalopentandra, Ceratosanthes, Citrullus, Coccinia, Corallocarpus, Cucumella, Cucumis, Cucurbita, Cyclantheropsis, Dendrosicyos, Doyera, Eureindra, Fevillea, Gerrandanthus, Gynostemma, Halosicyos, Ibervilla, Kedostris, Marah, Momordica, Neoalsomitra, Odosicyos, Parasicyos, Syrigia, Telfairia, Trochomeria, Trochomeriopsis, Tumamoca, Xerosicyos, Zehneria, Zygosicyos), Didiereaceae (en particulier les espèces: Alluaudia, Alluaudiopsis, Decaria, Didieara), Dioscoreaceae (en particulier les espèces: Dioscorea), Ericaceae (en particulier les espèces: Sphyrospermum), Eriospermaceae (en particulier les espèces: Eriospermum), Euphorbiaceae, Fabaceae (en particulier les espèces: Delonix, Dolichos, Erythrina, Neorautanenia, Pachyrhizus, Tylosema), Fouquieriaceae (en particulier les espèces: Fouquieria), Geraniaceae (en particulier les espèces: Monsonia, Pelargonium), Gesneriaceae (en particuler les espèces: Aeschynanthus, Alsobia, Chirita, Codonanthe, Columnea, Nematanthus, Sinningia, Streptocarpus), Hyacinthaceae (en particuler les espèces: Albuca, Bowiea, Dipcadi, Drimia, Drimiopsis, Hyacinthus, Lachenalia, Ledebouria, Litanthus, Massonia, Ornithogalum, Rhadamanthus, Rhodocodon, Schizobasis, Whiteheadia), Icacinaceae (en particuler les espèces: Pyrenacantha), Lamiaceae (en particuler les espèces: Aeollanthus, Dauphinea, Perrierastrum, Plectranthus, Solenostemon, Tetradenia, Thorncroftia), Lentibulariaceae, Loasaceae (en particuler les espèces: Schismocarpus), Loranthaceae (en particuler les espèces: Tapinanthus), Melastomataceae (en particuler les espèces: Medinilla), Meliaceae (en particuler les espèces: Entandrophragma), Menispermaceae (en particuler les espèces: Chasmanthera, Stephania, Tinospora), Moraceae (en particuler les espèces: Dorstenia, Ficus), Nolanaceae (en particuler les espèces: Nolana), Nolinaceae (en particuler les espèces: Beaucarnea, Calibanus, Dasylirion, Nolina), Orchidaceae, Oxalidaceae (en particuler les espèces: Oxalis), Passifloraceae (en particuler les espèces: Adenia), Pedaliaceae (en particuler les espèces: Pterodiscus, Sesamothamnus, Uncarina), Phyllanthaceae (en particuler les espèces: Phyllanthus), Phytolaccaceae (en particuler les espèces: Phytolacca), Piperaceae (en particuler les espèces: Peperomia), Portulacaceae (en particuler les espèces: Amphipetalum, Anacampseros, Avonia, Calyptrotheca, Ceraria, Cistanthe, Dendroportulaca, Grahamia, Lewisia, Parakeelya, Portulaca, Portulacaria, Schreiteria, Talinella, Talinum), Rubiaceae (en particuler les espèces: Anthorrhiza, Hydnophytum, Hydrophylax, Myrmecodia, Myrmephythum, Phylohydrax, Squamellaria), Ruscaceae (en particuler les espèces: Cordyline, Dracaena, Sansevieria), Sapindaceae (en particuler les espèces: Erythrophysa), Saxifragaceae, Sterculiaceae (en particuler les espèces: Brachychiton, Sterculia), Urticaceae (en particuler les espèces: Laportea, Obertia, Pilea, Sarcopilea), Viscaceae (en particuler les espèces: Viscum), Vitaceae(en particuler les espèces: Cissus, Cyphostemma), Xanthorrhoeaceae et Zygophyllaceae, dans un milieu de culture in vitro de manière à permettre une croissance des cellules, tout en les élicitant, ladite croissance et élicitation étant opérées en milieu in vitro sous une atmosphère gazeuse consistant en de l'air humide enrichi en CO2 avec une teneur de 1 à 5% en volume de CO2, et une humidité relative de plus de 50%, à une température comprise entre 15°C et 50°C selon un cycle comprenant 10 périodes d'obscurité ou de faible luminosité avec une luminosité de moins de 10 lux (en particulier de 1 à 5lux) pendant 20minutes et 3 heures, les périodes d'obscurité étant séparées l'une de l'autre par une période de luminosité dans la gamme de 100 à 700nm, de préférence de 400 à 520nm, de plus de 1000 lux pendant 1 à 6 heures (plus spécifiquement de plus de 10000 lux, de préférence de plus de 50000 lux et même plus particulièrement préférée de plus de 100000 lux), en ce que l'on cultive et élicite lesdites cellules végétales différenciées dans leur milieu de culture in vitro pendant un cycle suffisant long en temps pour la synthèse d'une quantité suffisante de métabolites dans les cellules dédifférentiées, en ce qu'on sépare au moins partiellement les cellules du milieu de culture, et en ce qu'on mélange les cellules végétales élicitées du milieu de culture avec un ou plusieurs excipients acceptables pour application topique pour préparer une composition topique, avantageusement cosmétique, selon l'invention.

Selon le procédé, l'atmosphère est de l'air enrichi en CO2 et en humidité si nécessaire.

Dans des formes de réalisation avantageuses du procédé selon l'invention, les cellules ont été avantageusement préparées en utilisant une ou des caractéristiques suivantes :
- le changement de luminosité lors du passage d'une période de luminosité inférieure à 10lux à une période de luminosité de plus de 1000lux (avantageusement plus de 50000lux) se fait en un temps de moins de 2 minutes.
- les cellules sont d'abord cultivées pour obtenir des cellules dédifférentiées, les cellules dédifférentiées ainsi obtenues sont mise en milieu de culture in vitro pour leur développement sous élicitation.

L'intérêt de ce procédé est qu'il permet d'obtenir des cellules végétales riches en molécules particulières (de la famile des stilbènes, flavanoïdes, néobétanine, alkaloïdes, vitamines, quercetine-3 méthyl éther, acides gras, dérivés de rutine ou rutinoside, acide galique, isorhamnetine, etc.) dans des proportions adéquates en grands volumes tout en répondant aux besoins de l'industrie, notamment :
- Le respect de la structure tertiaire des molécules,
- L'absence de solvant et de résidus,
- L'homogénéité des substrats,
- La production continue indépendamment du cycle des saisons,
- La conservation des caractéristiques biologiques et physiologiques sans ajout de conservateur,
- L'absence totale de polluants,
- La production standardisée et reproductible quant à la qualité et la concentration des métabolites,
- L'utilisation de ces suspensions végétales après lyophilisation directe avec utilisation de température inférieure à -30°C. Cette technique permet l'obtention d'une poudre très fine pouvant être dispersée dans des compositions cosmétiques (crèmes, pommades, lotions...). Ces cellules sont susceptibles de libérer directement les principes actifs qu'elles contiennent, sans passage par une extraction à l'aide de solvants organiques (élimination des risques de résidus).
- L'utilisation des extraits cellulaires uniquement après sonification et centrifugation.
- l'utilisation des cellules végétales dédifférenciées et élicitées fraiches, non broyées, mélangées à un excipient cosmétique (par exemple glycérine, glycol(s), huile(s)). Les cellules fraiches sont avanatgeusement isolées du milieu de culture, lavées et éventuellement rincées, avant d'être éventuellement séchées, avant d'être mélangées à un ou des excipients pour application cosmétique.

Cette technologie apporte une alternative utile et innovante aux extractions conventionnelles par solvants. La possibilité d'orienter de manière naturelle (élicitation) la synthèse des métabolites sans porter atteinte à l'intégrité génétique des cellules, représente une garantie de qualité et d'authenticité.

De manière tout à fait surprenante les inventeurs ont découvert que l'on pouvait directement incorporer ou disperser les cellules après élicitation, séchage dans une composition cosmétique et/ ou pharmaceutique. La composition selon l'invention contient alors de la matière des membranes de cellules. Ce procédé présente notamment l'avantage de désactiver les enzymes oxydants sans ajouts d'additifs ou de produits chimiques. Un autre aspect de l'invention permet de concentrer et de diriger la production de phytoalexines sans pertes quantitatives ou qualitatives dues aux extractions et aux filtrages.

Par composition à usage topique on entend des crèmes, des onguents, des lotions, suspensions, des bâtons, des shampooings, des gels, serums, laits, lotions, crèmes, des solutions (par exemple applicable par spray). La composition à usage topique est par exemple une composition cosmétique, dermatologique, une composition d'hygiène de la peau, un parfum, etc.

Préférentiellement selon l'invention, la composition est une composition cosmétique.

Des exemples et compositions suivants illustrent l'invention sans la limiter aucunement. Dans les compositions les proportions indiquées sont des pourcentages en poids.

Dans ces exemples, on a utilisé un procédé préféré tel que défini en résumé ci-après.

### Etape 1 : Préparation de cellules dédifférenciées et cultivés dans un milieu de culture in vitro

Cette étape de préparation de cellules dédifférenciées a été réalisée de manière classique. Pour cette étape on a utilisé des cellules végétales provenant de plantes appartenant aux familles suivantes :
Agavaceae (en particulier les espèces: Agave, Beschorneria, Chlorophytum, Furcraea, Hesperaloe, Hesperoyucca, Yucca), Amaryllidaceae (en particulier l'espèce: Boophane, Brunsvigia, Cyrtanthus, Haemanthus, Rauhia), Anacardiaceae (en particulier l'espèce: Operculicarya, Pachycormus), Apiaceae (en particulier l'espèce: Steganotaenia), Apocynaceae (en particulier l'espèce: Adenium, Mandevilla, Pachypodium, Plumeria), Araceae (en particulier les espèces: Zamioculcas zamiifolia); Araliaceae (en particulier les espèces: Cussonia), Asclepiadaceae (en particulier les espèces: Absolmsia, Asclepias, Aspidoglossum, Aspidonepsis, Baynesia, Brachystelma, Caralluma, Ceropegia, Cibirhiza, Cynanchum, Dischidia, Dischidiopsis, Duvalia, Duvaliandra, Echidnopsis, Edithcolea, Fanninia, Fockea, Glossostelma, Hoodia, Hoya, Huernia, Huerniopsis, Ischnolepis, Larryleachia, Lavrania, Madangia, Marsdenia, Matelea, Micholitzia, Miraglossum, Notechidnopsis, Odontostelma, Ophionella, Orbea, Orbeanthus, Pachycarpus, Pectinaria, Petopentia, Piaranthus, Pseudolithos, Quaqua, Raphionacme, Rhytidocaulon, Riocreuxia, Sarcorrhiza, Sarcostemma, Schizoglossum, Schlechterella, Stapelia, Stapelianthus, Stapeliopsis, Stathmostelma, Stenostelma, Stomatostemma, Tavaresia, Trachycalymma, Tridentea, Tromotriche, White-sloanea, Xysmalobium), Asparagaceae (en particulier les espèces: Myrsiphyllum), Asphodelaceae ((en particulier les espèces: Aloe, Astroloba, Bulbine, Chortolirion, Gasteria, Haworthia, Poellnitzia, Trachyandra), Asteraceae (en particulier les espèces: Baeriopsis, Coulterella, Crassocephalum, Didelta, Gynura, Osteospermum, Othonna, Polyachyrus, Pteronia, Senecio), Balsaminaceae (en particulier les espèces: Impatiens), Basellaceae (en particulier les espèces: Anredera, Basella), Begoniaceae (en particulier les espèces: Begonia), Bombacaceae (en particulier les espèces: Adansonia, Cavanillesia, Ceiba, Pseudobombax), Brassicaceae (en particulier les espèces: Heliophila, Lepidium), Bromeliaceae, Burseraceae (en particulier les espèces: Beiselia, Bursea, Commiphora), Cactaceae (en particulier les espèces: Acanthocalycium, Acanthocereus, Ariocarpus, Armatocereus, Arrojadoa, Arthrocereus, Astrophytum, Austrocactus, Aztekium, Bergerocactus, Blossfeldia, Brachycereus, Browningia, Brasilicereus, Calymmanthium, Carnegiea, Cephalocereus, Cephalocleistocactus, Cereus, Cintia, Cipocereus, Cleistocactus, Coleocephalocereus, Copiapoa, Corryocactus, Coryphantha, Dendrocereus, Denmoza, Discocactus, Disocactus, Echinocactus, Echinocereus, Echinopsis, Epiphyllum, Epithelantha, Eriosyce, Escobaria, Escontria, Espostoa, Espostoopsis, Eulychnia, Facheiroa, Ferocactus, Frailea, Geohintonia, Gymnocalycium, Haageocereus, Harrisia, Hatiora, Hylocereus, Jasminocereus, Lasiocereus, Leocereus, Lepismium, Leptocereus, Leuchtenbergia, Lophophora, Maihuenia, Malacocarpus, Mammillaria, Mammilloydia, Matucana, Melocactus, Micranthocereus, Mila, Monvillea, Myrtillocactus, Neobuxbaumia, Neolloydia, Neoraimondia, Neowerdermannia, Obregonia, Opuntia, Oreocereus, Oroya, Ortegocactus, Pachycereus, Parodia, Pediocactus, Pelecyphora, Peniocereus, Pereskia, Pereskiopsis, Pilosocereus, Polaskia, Praecereus, Pseudoacanthocereus, Pseudorhipsalis, Pterocactus, Pygmaeocereus, Quiabentia, Rauhocereus, Rebutia, Rhipsalis, Samaipaticereus, Schlumbergera, Sclerocactus, Selenicereus, Stenocactus, Stenocereus, Stephanocereus, Stetsonia, Strombocactus, Tacinga, Thelocactus, Turbinicarpus, Uebelmannia, Weberbauerocereus, Weberocereus, Yungasocereus), Campanulaceae (en particulier les espèces: Brighamia), Capparidaceae (en particulier les espèces: Maerua), Caricaceae (en particulier les espèces: Carica, Jacarathia), Chenopodiaceae, Cochlospermaceae, Commelinaceae (en particulier les espèces: Aneilema, Callisia, Cyanotis, Tradescantia, Tripogandra), Convolvulaceae (en particulier les espèces: Ipomea, Sictocardia, Turbina), Crassulaceae (en particulier les espèces: Adromischus, Aeonium, Afrovivella, Aichryson, Cotyledon, Crassula, Cremnophila, Cremnosedum, Dudleya, Echeveria, Graptopetalum, Hylotelephium, Hypagophytum, Kalanchoe, Lenophyllum, Meterostachys, Monanthes, Orostachys, Pachyphytum, Perrierosedum, Phedimus, Pistorinia, Prometheum, Pseudosedum, Rhodiola, Rosularia, Sedella, Sedum, Sempervivum, Sinocrassula, Thompsonella, Tylecodon, Umbilicus, Villadia), Cucurbitaceae (en particulier les espèces: Apodanthera, Brandegea, Cephalopentandra, Ceratosanthes, Citrullus, Coccinia, Corallocarpus, Cucumella, Cucumis, Cucurbita, Cyclantheropsis, Dendrosicyos, Doyera, Eureindra, Fevillea, Gerrandanthus, Gynostemma, Halosicyos, Ibervilla, Kedostris, Marah, Momordica, Neoalsomitra, Odosicyos, Parasicyos, Syrigia, Telfairia, Trochomeria, Trochomeriopsis, Tumamoca, Xerosicyos, Zehneria, Zygosicyos), Didiereaceae (en particulier les espèces: Alluaudia, Alluaudiopsis, Decaria, Didieara), Dioscoreaceae (en particulier les espèces: Dioscorea), Ericaceae (en particulier les espèces: Sphyrospermuin), Eriospermaceae (en particulier les espèces: Eriospermum), Euphorbiaceae, Fabaceae (en particulier les espèces: Delonix, Dolichos, Erythrina, Neorautanenia, Pachyrhizus, Tylosema), Fouquieriaceae (en particulier les espèces: Fouquieria), Geraniaceae (en particulier les espèces: Monsonia, Pelargonium), Gesneriaceae (en particuler les espèces: Aeschynanthus, Alsobia, Chirita, Codonanthe, Columnea, Nematanthus, Sinningia, Streptocarpus), Hyacinthaceae (en particuler les espèces: Albuca, Bowiea, Dipcadi, Drimia, Drimiopsis, Hyacinthus, Lachenalia, Ledebouria, Litanthus, Massonia, Ornithogalum, Rhadamanthus, Rhodocodon, Schizobasis, Whiteheadia), Icacinaceae (en particuler les espèces: Pyrenacantha), Lamiaceae (en particuler les espèces: Aeollanthus, Dauphinea, Perrierastrum, Plectranthus, Solenostemon, Tetradenia, Thorncroftia), Lentibulariaceae, Loasaceae (en particuler les espèces: Schismocarpus), Loranthaceae (en particuler les espèces: Tapinanthus), Melastomataceae (en particuler les espèces: Medinilla), Meliaceae (en particuler les espèces: Entandrophragma), Menispermaceae (en particuler les espèces: Chasmanthera, Stephania, Tinospora), Moraceae (en particuler les espèces: Dorstenia, Ficus), Nolanaceae (en particuler les espèces: Nolana), Nolinaceae (en particuler les espèces: Beaucarnea, Calibanus, Dasylirion, Nolina), Orchidaceae, Oxalidaceae (en particuler les espèces: Oxalis), Passifloraceae (en particuler les espèces: Adenia), Pedaliaceae (en particuler les espèces: Pterodiscus, Sesamothamnus, Uncarina), Phyllanthaceae (en particuler les espèces: Phyllanthus), Phytolaccaceae (en particuler les espèces: Phytolacca), Piperaceae (en particuler les espèces: Peperomia), Portulacaceae (en particuler les espèces: Amphipetalum, Anacampseros, Avonia, Calyptrotheca, Ceraria, Cistanthe, Dendroportulaca, Grahamia, Lewisia, Parakeelya, Portulaca, Portulacaria, Schreiteria, Talinella, Talinum), Rubiaceae (en particuler les espèces: Anthorrhiza, Hydnophytum, Hydrophylax, Myrmecodia, Myrmephythum, Phylohydrax, Squamellaria), Ruscaceae (en particuler les espèces: Cordyline, Dracaena, Sansevieria), Sapindaceae (en particuler les espèces: Erythrophysa), Saxifragaceae, Sterculiaceae (en particuler les espèces: Brachychiton, Sterculia), Urticaceae (en particuler les espèces: Laportea, Obertia, Pilea, Sarcopilea), Viscaceae (en particuler les espèces: Viscum), Vitaceae(en particuler les espèces: Cissus, Cyphostemma), Xanthorrhoeaceae et Zygophyllaceae.

Cette étape 1 est réalisée en chambre blanche, sous une atmosphère d'air, avec un éclairage constant de plus de 1000001ux, à une température de 30°C, et sous une humidité relative de 50%. Cette étape est opérée par repiquage successif d'une partie de la plante, en particulier d'une partie de la racine. L'éclairage était du type émettant à plus de 95% des rayons (en particulier à plus de 99%) dans la gamme 100nm à 700nm, de préférence 400 à 520 nm.

### Etape 2 : repiquage des cellules dédifférenciées de l'étape 1 dans un milieu de culture in vitro pour le développement et l'élicitation des cellules.

Le développement avec élicitation des cellules de l'étape 1 a été opéré dans un milei in vitro sous une atmosphère gazeuse contenant de l'oxygène et du CO2 selon un cycle comprenant 100 périodes de faible luminosité avec une luminosité de moins de 10 lux (de 1 à 5 lux) pendant 1 heure sous une atmosphère constitué d'air humide (humidité relative de 75%) enrichi de CO2 (pour que la teneur en CO2 soit de 5%) et présentant une température de 30°C, ces périodes de développement/élicitation sous faible (voire sans) luminosité (avantageusement obscurité sensiblement totale) étant séparées l'une de l'autre par une période de luminosité ou d'éclairage de plus de 100000 lux (L'éclairage était du type émettant à plus de 95% des rayons (en particulier à plus de 99%) dans la gamme 100nm à 700nm, de préférence 400 à 520 nm.) pendant 1 heure sous une atmosphère constituée d'air humide (humidité relative de 75%) enrichi de CO2 (pour que la teneur en CO2 de l'atmosphère soit de 5%) et présentant une température de 45°C. Le passage d'un état d'obscurité à un état éclairé est réalisé par le déplacement d'une paroi opaque. Cette culture est opérée en chambre blanche ou stérile ou asceptique.

On a remarqué que ce procédé de culture et élicitation des cellules permettait d'obtenir une teneur plus importante en phytoalexines, flavanoïdes (rutine, acide gallique, dérivés d'isorhamnetine, etc.) par rapport à celle obtenue par culture in vitro sous sous une atmosphère gazeuse contenant de l'oxygène et du CO2 selon un cycle d'éclairage permanent, mais même selon un cycle comprenant des périodes de faible luminosité avec une luminosité de moins de 10 lux (de 1 à 5 lux) pendant 12 à 24 heures sous une atmosphère constitué d'air humide (humidité relative de 75%) enrichi de CO2 (pour que la teneur en CO2 soit de 5%) et présentant une température de 30°C, ces périodes de faible (voire sans) luminosité étant séparées l'une de l'autre par une période de luminosité de plus de 100000 lux pendant 12 heures sous une atmosphère constituée d'air humide (humidité relative de 75%) enrichi de CO2 (pour que la teneur en CO2 de l'atmosphère soit de 5%) et présentant une température de 45°C.
On a également remarqué que l'enrichissement de l'atmosphère en CO2 avait un effet possitif sur la formation de phytoalexines, flavanoïdes, etc.

Etape 3 : Extraction des cellules dédifférenciées et élicitées en milieu de culture in vitro, par exemple par filtration du milieu de culture suivi d'une ou de plusieurs étapes de lavage (avec ou sans rinçage), en particulier opérées pour ne pas détruire la structure des membranes des cellules.

Les cellules dédifférenciées et élicitées en milieu de culture, lavées peuvent directement être mélangées à un ou des excipients cosmétiques (voir étape 6). Par exemple, les cellules fraiches sont mélangées à du glycérol et/ou un ou des glycols et/ou une ou des huiles (avantageusement du type végétale), la proportion en poids du mélange en cellules végétales étant avantageusement de 1 à 20%, ce mélange constituant alors un produit apte à être utilisé dans la préparation d'une composition cosmétique.

Etape 4 éventuelle, mais avantageuse : Séchage ou lyophilisation des cellules dédifférenciées et élicitées en milieu de culture in vitro, cette opération de séchage étant avantageusement opérée pour ne pas détruire la structure des membranes des cellules.

Cette étape est avantageusement opérée à une température inférieure à 60° C, par exemple entre -60° C et 50°C.

Etape 5 : mélange et / ou incorporation à un ou des excipients et/ ou à d'autres principes actifs (notamment d'autres cellules/ broyats végétaux) pour la préparation de la composition à usage topique. Par exemple mélange des cellules végétales fraiches dans du glycérol et/ou un ou des glycols et/ou dans une ou des huiles pour obtenir une composition prête à être utilisée pour la préparation d'une composition cosmétique topique par ajout d'un ou d'excipients supplémentaires.

### Exemple d'Activité pharmacologique antioxydante

L'activité anti-radicalaire des cellules végétales produits par le procédé décrit ci-avant a été étudiée in vitro, en utilisant un modèle d'épidermes reconstitués SKINETHIC®, permettant de révéler cette activité par dosage du malondialdéhyde (MDA), après son induction par des rayons ultraviolets B.

En conclusion, les cellules obtenues par le procédé selon l'invention présente(nt) un effet anti-radicalaire aussi bien dans des conditions physiologiques que dans les conditions d'induction par les rayonnements ultraviolets B. Il ressort de cet essai un effet anti-radicalaire significatif.

### Exemple de Dispersion de cellules élicitées dans une base cosmétique

Les cellules végétales préparées comme décrit ci-avant sont utilisées pour la préparation d'une composition cosmétique. Les cellules sont dispersées après lyophilisation sans avoir été broyées dans la base suivante :

| | |
|---|---|
| - eau déminéralisée | 85,61 % |
| - huile minérale | 9,00 % |
| - alcool cétylique | 3,00 % |
| - ceteareth - 20 | 0,75 % |
| - cellules végétales (non broyées) | 0,20 % |
| - parfum | 0,15 % |
| - carbomer | 0,10 % |
| - methylchloroisothiazoline et methylisothiazoline [kathon CG] | 0,065 % |
| - hydroxyde de sodium (45 %) | 0,06 % |
| - hydroxyanisole de butyle | 0,06 % |
| | |
| TOTAL | 100,00 % |

La composition obtenue montre une dispersion homogène des cellules dans la crème et une granulométrie très fine. L'étude de propreté a montré l'absence de germes et champignons ainsi qu'une remarquable stabilité de la composition. Le résultat obtenu ayant été testé dans une étude transcutanée a permis de voir le passage des principes actifs notamment les polyphénols et autres flavanoïdes à travers le tissu cutané.

Les cellules végétales (non broyées) peuvent être utilisées dans des crèmes, lotions, shampoingss, gels, solutions, laits

La proportion des cellules, sous forme d'une suspension visqueuse ou d'un gel ou d'une poudre sensiblement sèche est fonction de la nature de la composition à usage topique et de l'application souhaitée. Elle est avantageusement comprise entre 0,01 et 5 %, mais peut atteindre jusqu'à 25 %.

Evidemment, l'invention n'est pas limitée aux exemples de réalisation donnés ci-dessus et il est possible de réaliser la composition à usage topique sous d'autres formes, telles que huile, onguent, laques, fards (fond de teint, poudre, rouge à lèvres, crayon, mascara, ombre à paupières) qui entrent aussi dans le cadre de l'invention.

Des compositions cosmétiques selon l'invention (avec une teneur de 1% de broyat de cellules végétales - sous forme sèche) ont été testées sur des volontaires. On a remarqué que de telles compositions avait un effet anti-âge, un effet protecteur de la peau, un effet anti oxydant, antiradicalaire, anti fongique, anti acné, de régénation de la peau, etc.

## Revendications

1. Procédé de préparation d'une composition à usage topique comprenant un matériau issu de cellules végétales d'une espèce choisie parmi le groupe constitué des espèces des familles suivantes: Agavaceae, Amaryllidaceae, Anacardiaceae, Apiaceae, Apocynaceae, Araceae; Araliaceae, Asclepiadaceae, Asparagaceae, Asphodelaceae, Asteraceae, Balsaminaceae, Begoniaceae, Bombacacea, Brassicaceae, Bromeliaceae, Burseraceae, Cactaceae, Campanulaceae, Capparidaceae, Caricaceae, Chenopodiaceae, Cochlospermaceae, Commelinaceae, Convolvulaceae, Crassulaceae, Cucurbitaceae, Didiereaceae, Dioscoreaceae, Ericaceae, Eriospermaceae, Euphorbiaceae, Fabaceae, Fouquieriaceae, Geraniaceae, Gesneriaceae, Hyacinthaceae, Icacinaceae, Lamiaceae, Lentibulariaceae, Loasaceae, Loranthaceae, Melastomataceae, Meliaceae, Menispermaceae, Moraceae, Nolanaceae, Nolinaceae, Orchidaceae, Oxalidaceae, Passifloraceae, Pedaliaceae, Phyllanthaceae, Phytolaccaceae, Piperaceae, Portulacaceae, Rubiaceae, Ruscaceae, Sapindaceae, Saxifragaceae, Sterculiaceae, Urticaceae, Viscaceae, Vitaceae, Xanthorrhoeaceae et Zygophyllaceae, **caractérisé en ce que** l'on met des cellules végétales dédifférentiées dans un milieu de culture in vitro de manière à permettre une croissance des cellules, tout en les élicitant, ladite croissance et élicitation étant opérées en milieu in vitro sous une atmosphère gazeuse consistant en de l'air humide enrichi en CO₂ avec une teneur de 1 à 5 % en volume de CO₂, et une humidité relative de plus de 50%, à une température comprise entre 15°C et 50°C selon un cycle comprenant au moins 10 périodes d'obscurité ou de faible luminosité avec une luminosité de moins de 10 lux pendant 20minutes à 3 heures, les périodes d'obscurité ou de faible luminosité étant séparées l'une de l'autre par une période de luminosité dans la gamme de 100 à 700nm, de préférence de 400 à 520nm, de plus de 1000lux, pendant 1 à 6 heures, **en ce que** l'on cultive et élicite lesdites cellules végétales différenciées dans leur milieu de culture pendant ce cycle suffisant long en temps pour la synthèse d'une quantité suffisante de métabolites dans les cellules dédifférentiées élicitées, **en ce qu'**on sépare au moins partiellement les cellules du milieu de culture, et **en ce qu'**on mélange les cellules végétales dédifférentiées et élicitées non broyées séparées au moins partiellement du milieu de culture avec un ou plusieurs excipients acceptables pour application topique pour préparer la composition topique.

2. Procédé suivant la revendication 1, **caractérisé en ce que** l'atmosphère contient 5% en volume de CO₂.

3. Procédé suivant l'une quelconque des revendications 1 et 2, **caractérisé en ce que** le cycle comprend au moins dix périodes d'obscurité ou de faible luminosité séparées l'une de l'autre par une période de luminosité dans la gamme de 400 à 520nm, de plus de 10000lux.

4. Procédé suivant l'une quelconque des revendications 1 à 3, **caractérisé en ce que** le cycle comprend au moins dix périodes d'obscurité ou de faible luminosité séparées l'une de l'autre par une période de luminosité de plus de 100000lux.

5. Procédé suivant l'une quelconque des revendications 1 à 4, **caractérisé en ce que** le passage d'une période d'obscurité ou de luminosité inférieure à 10lux à une période de luminosité de plus de 1000 lux se fait en un temps de moins de 2 minutes.

6. Procédé suivant l'une quelconque des revendications 1 à 5, **caractérisé en ce que** le passage d'une période d'obscurité ou de luminosité inférieure à 10lux à une période de luminosité de plus de 50000 lux se fait en un temps de moins de 2 minutes.

7. Procédé suivant l'une quelconque des revendications 1 à 6, **caractérisé en ce que** les cellules végétales ont été élicitées en milieu in vitro sous atmosphère comprenant de l'eau pour obtenir une humidité relative proche de la saturation.

8. Procédé suivant la revendication 1, **caractérisé en ce que** le développement avec élicitation des cellules est opéré selon un cycle comprenant 100 périodes d'obscurité ou de faible luminosité de moins de 10lux pendant 1 heure sous une atmosphère constituée d'air humide avec une humidité relative de 75% et enrichi de CO₂pour que sa teneur soit de 5% et présentant une température de 30°C, les périodes d'obscurité ou de faible luminosité étant séparées l'une de l'autre par une période de luminosité de plus de 100000 lux émettant à plus de 95% des rayons dans la gamme 100nm à 700nm, pendant 1 heure sous une atmosphère constituée d'air humide avec une humidité relative de 75% et enrichi de CO₂ pour que sa teneur soit de 5% et présentant une température de 45°C.

9. Procédé suivant la revendication 8, **caractérisé en ce que** l'éclairage des périodes de luminosité de plus de 100000 lux est opéré au moyen d'un éclairage émettant plus de 99% des rayons dans la gamme 400nm à 520nm.

## Patentansprüche

1. Verfahren zur Herstellung einer Zusammensetzung zur topischen Verwendung, umfassend ein Material aus Pflanzenzellen einer Art, die aus der Gruppe bestehend aus den Arten der folgenden Familien ausgewählt wird: Agavaceae, Amaryllidaceae, Anacardiaceae, Apiaceae, Apocynaceae, Araceae; Araliaceae, Asclepiadaceae, Asparagaceae, Asphodelaceae, Asteraceae, Balsaminaceae, Begoniaceae, Bombacaceae, Brassicaceae, Bromeliaceae, Burseraceae, Cactaceae, Campanulaceae, Capparidaceae, Caricaceae, Chenopodiaceae, Cochlospermaceae, Commelinaceae, Convolvulaceae, Crassulaceae, Cucurbitaceae, Didiereaceae, Dioscoreaceae, Ericaceae, Eriospermaceae, Euphorbiaceae, Fabaceae, Fouquieriaceae, Geraniaceae, Gesneriaceae, Hyacinthaceae, Icacinaceae, Lamiaceae, Lentibulariaceae, Loasaceae, Loranthaceae, Melastomataceae, Meliaceae, Menispermaceae, Moraceae, Nolanaceae, Nolinaceae, Orchidaceae, Oxalidaceae, Passifloraceae, Pedaliaceae, Phyllanthaceae, Phytolaccaceae, Piperaceae, Portulacaceae, Rubiaceae, Ruscaceae, Sapindaceae, Saxifragaceae, Sterculiaceae, Urticaceae, Viscaceae, Vitaceae, Xanthorrhoeaceae und Zygophyllaceae, **dadurch gekennzeichnet, dass** die dedifferenzierten Pflanzenzellen in ein Kulturmedium in vitro eingebracht werden, um ein Wachstum von Zellen zu ermöglichen, wobei sie gleichzeitig angeregt werden, wobei das Wachstum und das Anregen in dem Medium in vitro unter einer Gasatmosphäre, die aus mit CO₂angereicherter feuchter Luft mit einem Gehalt von 1 bis 5 Vol.-% CO₂ und einer relativen Feuchtigkeit von mehr als 50% besteht, bei einer Temperatur zwischen 15°C und 50°C gemäß einem Zyklus vorgenommen werden, der mindestens 10 Dunkel oder Schwachlichtperioden mit einer Helligkeit von weniger als 10 Lux während 20 Minuten bis 3 Stunden umfasst, wobei die Dunkel- oder Schwachlichtperioden voneinander durch eine Helligkeitsperiode im Bereich von 100 bis 700nm, vorzugsweise von 400 bis 520nm, von mehr als 1000 Lux, während 1 bis 6 Stunden getrennt werden, dass die genannten differenzierten Pflanzenzellen in ihrem Kulturmedium während dieses Zyklus während einer ausreichend langen Zeit für die Synthese einer ausreichenden Menge an Metaboliten in den angeregten dedifferenzierten Zellen kultiviert und angeregt werden, dass die Zellen von dem Kulturmedium mindestens teilweise abgetrennt werden und dass die dedifferenzierten und angeregten Pflanzenzellen, die nicht zerkleinert und mindestens teilweise von dem Kulturmedium getrennt sind, mit einem oder mehreren annehmbaren Exzipienten zur topischen Anwendung zur Herstellung der topischen Zusammensetzung gemischt werden.

2. Verfahren nach Anspruch 1, **dadurch gekennzeichnet, dass** die Atmosphäre 5 Vol.-% CO₂ enthält.

3. Verfahren nach einem der Ansprüche 1 und 2, **dadurch gekennzeichnet, dass** der Zyklus mindestens zehn Dunkel- oder Schwachlichtperioden umfasst, die voneinander durch eine Helligkeitsperiode im Bereich von 400 bis 520nm von mehr als 10000 Lux getrennt werden.

4. Verfahren nach einem der Ansprüche 1 bis 3, **dadurch gekennzeichnet, dass** der Zyklus mindestens zehn Dunkel- oder Schwachlichtperioden umfasst, die voneinander durch eine Helligkeitsperiode von mehr als 100000 Lux getrennt werden.

5. Verfahren nach einem der Ansprüche 1 bis 4, **dadurch gekennzeichnet, dass** der Übergang von einer Dunkel- oder Schwachlichtperiode von weniger als 10 Lux zu einer Helligkeitsperiode von mehr als 1000 Lux in einer Zeit von weniger als 2 Minuten erfolgt.

6. Verfahren nach einem der Ansprüche 1 bis 5, **dadurch gekennzeichnet, dass** der Übergang von einer Dunkel- oder Schwachlichtperiode von weniger als 10 Lux zu einer Helligkeitsperiode von mehr als 50000 Lux in einer Zeit von weniger als 2 Minuten erfolgt.

7. Verfahren nach einem der Ansprüche 1 bis 6, **dadurch gekennzeichnet, dass** die Pflanzenzellen in einem Medium in vitro unter einer Atmosphäre angeregt wurden, die Wasser umfasst, um eine relative Feuchtigkeit nahe bei der Sättigung zu erhalten.

8. Verfahren nach Anspruch 1, **dadurch gekennzeichnet, dass** die Entwicklung mit der Anregung der Zellen gemäß einem Zyklus vorgenommen wird, der 100 Dunkel- oder Schwachlichtperioden von weniger als 10 Lux während 1 Stunde unter einer Atmosphäre umfasst, die aus feuchter Luft mit einer relativen Feuchtigkeit von 75% und angereichert mit CO₂, damit ihr Gehalt 5% beträgt, und mit einer Temperatur von 30°C besteht, wobei die Dunkel oder Schwachlichtperioden voneinander getrennt werden durch eine Helligkeitsperiode von mehr als 100000 Lux, wobei über 95% der Strahlen in dem Bereich von 100nm bis 700nm emittiert werden, während 1 Stunde unter einer Atmosphäre, die aus feuchter Luft mit einer relativen Feuchtigkeit von 75% und angereichert mit CO₂, damit ihr Gehalt 5% beträgt, und mit einer Temperatur von 45°C besteht.

9. Verfahren nach Anspruch 8, **dadurch gekennzeichnet, dass** die Beleuchtung der Helligkeitsperioden von mehr als 100000 Lux mit Hilfe einer Beleuchtung vorgenommen wird, die mehr als 99% der Strahlen in dem Bereich von 400nm bis 520nm emittiert.

## Claims

1. A method of preparing a composition for topical use comprising a material derived from plant cells of a species chosen from the group consisting of species of the following families: Agavaceae, Amaryllidaceae, Anacardiaceae, Apiaceae, Apocynaceae, Araceae; Araliaceae, Asclepiadaceae, Asparagaceae, Asphodelaceae, Asteraceae, Balsaminaceae, Begoniaceae, Bombacacea, Brassicaceae, Bromeliaceae, Burseraceae, Cactaceae, Campanulaceae, Capparidaceae, Caricaceae, Chenopodiaceae, Cochlospermaceae, Commelinaceae, Convolvulaceae, Crassulaceae, Cucurbitaceae, Didiereaceae, Dioscoreaceae, Ericaceae, Eriospermaceae, Euphorbiaceae, Fabaceae, Fouquieriaceae, Geraniaceae, Gesneriaceae, Hyacinthaceae, Icacinaceae, Lamiaceae, Lentibulariaceae, Loasaceae, Loranthaceae, Melastomataceae, Meliaceae, Menispermaceae, Moraceae, Nolanaceae, Nolinaceae, Orchidaceae, Oxalidaceae, Passifloraceae, Pedaliaceae, Phyllanthaceae, Phytolaccaceae, Piperaceae, Portulacaceae, Rubiaceae, Ruscaceae, Sapindaceae, Saxifragaceae, Sterculiaceae, Urticaceae, Viscaceae, Vitaceae, Xanthorrhoeaceae and Zygophyllaceae, **characterized in that** dedifferentiated plant cells are placed in an in vitro culture medium so as to allow cell growth, while eliciting them, said growth and elicitation being carried out in an in vitro medium under a gaseous atmosphere consisting of humid air enriched in CO₂ with a content of 1 to 5% by volume of CO₂, and a relative humidity of more than 50 %, at a temperature between 15°C and 50°C according to a cycle comprising at least 10 periods of darkness or low light with a brightness of less than 10 lux for 20 minutes to 3 hours, periods of darkness or low light being separated from each other by a light period in the range of 100 to 700nm, preferably 400 to 520nm, of more than 1000lux, for 1 to 6 hours, **in that** said differentiated plant cells are growth and elicited in their culture medium during this cycle for a sufficient time for the synthesis of a sufficient quantity of metabolites in the elicited dedifferentiated cells, **in that** the cells are at least partially separated from the culture medium, and **in that** the uncrushed dedifferentiated and elicited cells at least partially separated from the culture medium are mixed with one or more excipients acceptable for topical application to prepare the topical composition.

2. Method according to claim 1, **characterized in that** the atmosphere contains 5% by volume of CO₂.

3. Method according to any one of claims 1 and 2, **characterized in that** the cycle comprises at least ten periods of darkness or low light separated from each other by a period of light in the range of 400 at 520nm, of more than 10000lux.

4. Method according to any one of claims 1 to 3, **characterized in that** the cycle comprises at least ten periods of darkness or low light separated from each other by a light period of more than 100000lux.

5. Method according to any one of claims 1 to 4, **characterized in that** the passage from a period of darkness or of brightness of less than 10lux to a period of light of more than 1000 lux takes place in a time of less than 2 minutes.

6. Method according to any one of claims 1 to 5, **characterized in that** the passage from a period of darkness or of brightness of less than 10lux to a period of light of more than 50000 lux takes place in a time of less than 2 minutes.

7. Method according to any one of claims 1 to 6, **characterized in that** the plant cells were elicited in an in vitro medium under an atmosphere comprising water to obtain a relative humidity close to saturation.

8. Method according to claim 1, **characterized in that** the growth with elicitation of the cells is carried out according to a cycle comprising 100 periods of darkness or low light of less than 10lux for 1 hour under an atmosphere consisting of humid air with a relative humidity of 75% and enriched with CO₂ so that its content is 5% and having a temperature of 30°C, the periods of darkness or low light being separated from each other by a period of light of more than 100000 lux emitting more than 95% of the rays in the range 100nm to 700nm, for 1 hour under an atmosphere consisting of humid air with a relative humidity of 75% and enriched with CO₂ so that its content is 5% and having a temperature of 45°C.

9. Method according to claim 8, **characterized in that** the lighting of the periods of light of more than 100000 lux is operated by means of lighting emitting more than 99% of the rays in the range of 400nm to 520nm.
